# EUROPÄISCHE PATENTSCHRIFT

(11) **EP 2 296 587 B1**
(45) Veröffentlichungstag und Bekanntmachung des Hinweises auf die Patenterteilung: **12.09.2018**
(21) Anmeldenummer: 09771887.8
(22) Anmeldetag: 09.06.2009
(51) Int. Cl.: A61F 2/82

(54) **METALLISCHER STENT ZUR BEHANDLUNG VON LÄSIONEN IN BLUTGEFÄSSEN MIT EINER VERPACKUNG**
METAL STENT FOR TREATING LESIONS IN BLOOD VESSELS, COMPRISING A PACKAGING
STENT MÉTALLIQUE POUR LE TRAITEMENT DE LÉSIONS DANS DES VAISSEAUX SANGUINS COMPORTANT UNE ENVELOPPE

(30) Priorität: 04.07.2008 CH 10362008
(43) Veröffentlichungstag der Anmeldung: 23.03.2011
(73) Patentinhaber: Qvanteq AG, 8005 Zürich (CH)
(72) Erfinder: Zucker, Arik, 8003 Zürich (CH)
(74) Vertreter: Latscha Schöllhorn Partner AG
(86) Internationale Anmeldenummer: PCT/CH2009/000190
(87) Internationale Veröffentlichungsnummer: WO 2010/000080

(56) Entgegenhaltungen:
- WO-A-00/44305
- WO-A-99/38546
- WO-A-03/030957
- WO-A-2005/073091
- WO-A-2008/021481
- US-A1- 2004 088 043
- US-A1- 2007 084 144
- US-A1- 2008 011 640

## Beschreibung

### Anwendungsgebiet der Erfindung

Die vorliegende Erfindung betrifft eine Anordnung, bestehend aus einem metallischen Stent als medizinisches Implantat zur Behandlung von Läsionen in Blutgefässen und einer Verpackung. Im Innenvolumen der Verpackung ist der Stent geschützt angeordnet. Der Stent hat eine Vielzahl von Stegen, die miteinander eine Röhrenform bilden. Zwischen dem proximalen und dem distalen Ende erstrecken sich die Stentlänge und als Durchgang das Stentlumen. Im dilatierten bzw. freigesetzten Zustand nimmt der Stent einen entsprechenden Durchmesser ein. Zur Begünstigung der Hämokompatibilität ist die Stentoberfläche hydrophil ausgebildet.

Ein spezieller Anwendungsbereich ist die Gefässdilatation im Gebiet der perkutanen transluminalen Angioplastie, darunter auch kardiovaskuläre Intervention. Derartige Stents werden mit einem speziell dafür vorgesehenen Katheter durch eine minimale Öffnung, z.B. mittels Arterienpunktion im Oberschenkelbereich in den menschlichen Körper bis zur Läsion, d.h. der zu behandelnden Gefässverengung, eingeführt und dort dilatiert. Während der Stent im dilatierten Blutgefäss verbleibt und dieses von innen her stützt, wird der Katheter aus dem Körper entfernt. Der Blutfluss ist durch das dilatierte und unterstützte Gefäss wieder gewährleistet. Dieser Vorgang wird mit Hilfe von instantanen Röntgenaufnahmen durchgeführt, welche sowohl die Blutgefässe, als auch die in den Körper eingeführten Instrumente auf einem Monitor darstellen.

Ein anderer spezieller Anwendungsbereich ist die Behandlung von Aneurysmen, d.h. erweiterten Blutgefässen. Bei dieser Behandlung wird ein Stent Graft - bestehend aus einem Stützgeflecht und einer Ummantelung - in das Aneurysma eingesetzt, um den herkömmlichen Blutfluss wieder zu gewährleisten.

### Stand der Technik

Die in Blutgefässen implantierten metallischen Stents bergen jedoch gewisse Risiken für den Patienten. Unter anderem können sich Thrombosen an den Strukturen des Stents bilden. In Kombination von Medikamenten, welche man den Patienten nach der Implantation verabreichte, konnten die Fälle von Thrombosen bei "nackten" Metall-Stents (Bare Metal Stents, BMS) unter 1% innerhalb der ersten 10 Tage gesenkt werden. Dennoch ist dies eine der meist gefürchteten Komplikationen, insbesondere bei der Koronarintervention.

Ein von den Medizinern gewünschtes Verhalten der Stents ist ein raschmögliches Einwachsen, die sogenannte Reendothelialisierung. Dies ist äusserst wichtig für den Erfolg der Stent-Therapie, weil die Zellen in dieser Endothelschicht essentielle antithrombotische Faktoren bilden. Solange der Stent jedoch noch nicht eingewachsen ist, und seine Strukturen dem Blutfluss ausgesetzt sind, ist es äusserst wichtig, eine antithrombogene Stentoberfläche zu haben.

Es ist bekannt, dass Stents mit hydrophilen Oberflächeneigenschaften eine sehr viel höhere Hämokompatibilität, d.h. eine viel tiefere Thrombogenizität, aufweisen. Demgemäss ist etwa aus der WO 2008/021481 A2 ein Stent bekannt, der aus hydrophilem Material, wie z. B. Metall oder Cellulose, gefertigt ist. Der Stent wird in einer Verpackung gelagert und kann vor dem Verpacken oder gemeinsam mit der Verpackung sterilisiert werden, um die Reinheit des Stents sicherzustellen. Um die Hydrophilizität auf den Stentoberflächen zu erhöhen, hat man mittels Beschichtungsverfahren Substanzen auf die Stentoberfläche aufgetragen [vgl. Seeger JM, Ingegno MD, Bigatan E, Klingman N, Amery D, Widenhouse C, Goldberg EP. Hydrophilic surface modification of metallic endoluminal stents. J Vasc Surg. 1995 Sep;22(3):327-36; Lahann J, Klee D, Thelen H, Bienert H, Vorwerk D, Hocker H. Improvement of haemocompatibility of metallic stents by polymer coating. J Mater Sci Mater Med. 1999 Jul;10(7):443-8].

Mögliche Beschichtungsverfahren sind beispielsweise "chemical vapor deposition" (CVD) oder "physical vapor deposition" (PVD), durch welche Materialien, wie z.B. Polymere oder Metalle in definierten Schichtdicken auf die Stentoberfläche aufgetragen werden. An einem mit Polymer beschichteten BMS ergab sich, dass durch die erhöhten hydrophilen Eigenschaften der Oberfläche die Plättchenbildung von 85% (BMS) auf 20% (Polymerbeschichteter BMS) reduziert wurde.

Während des klinischen Eingriffs treten einerseits starke, auf die Stentoberfläche einwirkende Reibungskräfte auf, andererseits werden während dem Aufdehnen hohe mechanische Spannungen auf der Oberfläche der einzelnen Stent-Stegen erzeugt. Nach der Implantation ist der Stent einer vom Blutgefäss ausgehenden dauerhaften, pulsierenden Belastung ausgesetzt. Durch diese hohen mechanischen Belastungen kann es zur Ablösung der Beschichtung kommen, wodurch ein erhebliches potentielles Risiko von Thrombosen, Mikroembolien aus Beschichtungspartikeln und schwerwiegenden chronischen Entzündungen entsteht. Zudem wurden selbst an noch nicht implantierten Stents kritische Unregelmässigkeiten in deren Beschichtung festgestellt.

Nebst den mechanischen Einflüssen werden die Stentbeschichtungen durch die im Körper auftretenden chemischen Reaktionen beschädigt oder abgebaut. Metallische Beschichtungen können korrodieren sobald das unterschiedliche elektrochemische Potential zwischen Beschichtung und Stent durch einen Elektrolyten - z.B. Blut - über den Batterie-Effekt ausgeglichen werden kann.

Polymerbeschichtungen auf Stents werden sukzessive vom Körper durch Enzyme abgebaut. Dieser Vorgang ist oft mit einer Entzündung der umliegenden Gefässzellen verbunden, welche ungewünschte Zellwucherungen, sogenannte Proliferationen, hervorrufen, was zu einer Wiederverengung (Restenose) des Blutgefässes führen kann. Zudem können Entzündungen bereits durch die Polymerbeschichtung selbst hervorgerufen werden.

Derartige Oberflächenmodifikationen begünstigen zwar - als positiver Effekt - das Einwachsverhalten von Stents, können jedoch aufgrund der vorgenannten Probleme klinische Komplikationen verursachen. Bis anhin steht kein Stent mit einer optimalen, hydrophilen Oberfläche zur Verfügung, welche sowohl den medizinischen, als auch den mechanischen Anforderungen genügt.

### Aufgabe der Erfindung

Angesichts der bisherigen Nachteile im Stand der Technik, liegt der Erfindung die Aufgabe zugrunde, einen Stent zu schaffen, welcher durch Oberflächenmodifikation eine erhöhte Hydrophilizität und Biokompatibilität aufweist und somit die vorgenannten Probleme vermeidet. Zugleich ist eine Verpackung für die Lagerung und Transport des mit der Oberflächenmodifikation versehenen Stents zu schaffen, um die hydrophilen Oberflächeneigenschaften des Stents bis zu seiner Intervention aufrechtzuerhalten.

Eine weitere Aufgabe besteht darin, bei der Lagerung von zunächst ungecrimpten Stents in der Verpackung, Mittel für die Montage des Stent auf einen Katheter vorzuschlagen, wobei die hydrophilen Eigenschaften der Stentoberfläche erhalten bleiben müssen.

Diese Aufgaben werden durch eine Anordnung bestehend aus einem metallischen Stent und einer Verpackung gemäss Patentanspruch 1 gelöst. Besondere Ausführungsformen dieser Anordnung gehen aus den Unteransprüchen hervor.

### Übersicht über die Erfindung

Die erfindungsgemässe Anordnung besteht aus einem metallischen Stent, als medizinisches Implantat zur Behandlung von Läsionen in Blutgefässen, und einer Verpackung, mit einem Innenvolumen, in welchem der Stent geschützt angeordnet ist. Der Stent hat eine Vielzahl von Stegen, die miteinander eine Röhrenform bilden, sowie ein proximales Ende und ein distales Ende, zwischen denen sich ein Stentlumen erstreckt. Die Stentoberfläche besitzt eine hydrophile Eigenschaft. Die aus der Atmosphäre stammenden molekularen chemischen Verunreinigungen, vorrangig Kohlenwasserstoff-Verbindungen, sind auf der Oberfläche durch eine Behandlung signifikant reduziert, wodurch sich der Kontaktwinkel als Mass der Hydrophilizität eines an der Oberfläche aufsitzenden Wassertropfens im Verhältnis zum Kontaktwinkel vor dieser Behandlung verringert. Der Stent ist in der Verpackung inert gelagert, um eine natürliche Rekontamination aus der Atmosphäre zu verhindern.

Die nachfolgenden Merkmale beziehen sich auf spezielle Ausführungen der Erfindung: Die Behandlung der Oberfläche zur Reduktion der chemischen Verunreinigungen ist als Materialabtrag erfolgt, nämlich z.B. mittels Sputtern als Beschuss mit Ionen, Elektroerosion, Elektropolieren, Plasmaaktivieren, Laserablation, mechanisch abrasivem Verfahren, Trockenätzen oder nass-chemisch Ätzen.

Alternativ hat die Behandlung der Oberfläche zur Reduktion der chemischen Verunreinigungen eine unveränderte Topographie der Oberfläche als Ergebnis, wobei auch hier die Behandlung z.B. mittels Sputtern als Beschuss mit Ionen, Elektroerosion, Elektropolieren, Plasmaaktivieren, Laserablation, mechanisch abrasivem Verfahren, Trockenätzen oder nass-chemisch Ätzen erfolgt ist. Eine nicht material-abtragende Behandlung, z.B. mittels Ultraschall, UV-Licht oder Ozon oder einer daraus gebildeten Kombinationsbehandlung, kann ebenfalls zu einer unveränderten Oberflächentopographie führen. Gleichermassen ist hierfür ein Ätzmedium geeignet, welches das Stentmaterial selbst nicht angreift.

Der gesamte Inhalt der Verpackung ist inert und die Verpackung enthält eine inerte Füllung.

In der Verpackung ist ein Katheter angeordnet, auf dem ein Stent montiert ist, wobei einem ballon-expandierenden bzw. einem selbst-expandierenden Stent komplementär ein Ballonkatheter bzw. ein Schlauchkatheter zugeordnet ist.

Die Verpackung besteht aus einem Behältnis mit einem Boden und einem Deckel. Der Boden und/oder der Deckel lassen sich entfernen. Der Boden und/oder der Deckel weisen einen zu öffnenden Zugang auf, so dass sich der Stent aus der Verpackung entnehmen lässt, bzw. der auf einem Katheter montierte Stent sich zusammen mit dem Katheter aus der Verpackung entnehmen lässt.

Der Katheter hat an seinem distalen Ende eine Spitze, und das der Spitze gegenüber liegende proximale Ende des Schafts des Katheters durchragt den Zugang nach ausserhalb der Verpackung.

Im Boden oder im Deckel ist ein Durchgang zum Durchlass einer Welle vorhanden, welche nach innen in die Verpackung zu den Backen einer integrierten Crimpvorrichtung und nach aussen zu einem Aktivator zur Betätigung der Crimpvorrichtung führt. Dem Durchgang gegenüberliegend im Deckel bzw. im Boden ist der zu öffnende Zugang vorhanden, der dem Durchlass eines Katheters dient. Axial durch die Crimpvorrichtung erstreckt sich ein Führungsdorn, welcher zur Stabilisierung und Positionierung nach dem vollständigen Einführen in ein Führungsdrahtlumen des Katheters bestimmt ist. Der zu öffnende Zugang ist vorteilhaft z.B. aus einer durchdringbaren Dichtung oder aus einem perforierbaren Material beschaffen.

In der Verpackung erstrecken sich Stützelemente zur Fixierung des Stents und/oder des Katheters und/oder der Crimpvorrichtung.

Für die Anwendung bei Aneurysmen ist der Stent mit einer Ummantelung zu einem Stent Graft ausgebildet.

### Kurzbeschreibung der beigefügten Zeichnungen

Es fallen alle im weiteren gezeigten Beispiele unter die Erfindung. Es zeigen:
- Figur 1A:: einen Stent, ballon- oder selbst-expandierend, im nichtgecrimpten Zustand;
- Figur 1B:: Verpackung mit darin in einer Inertfüllung gelagertem Stent gemäss Figur 1A;
- Figur 2A:: die Anordnung gemäss Figur 1B mit Zugang in die Verpackung und darin integrierter offener Crimpvorrichtung;
- Figur 2B:: die Crimpvorrichtung aus Figur 2A im offenen Zustand;
- Figur 2C:: die Crimpvorrichtung gemäss Figur 2B im geschlossenen Zustand;
- Figur 3A:: die Anordnung gemäss Figur 2A, mit einem ballon-expandierenden Stent, mit einem in Position gebrachten Dilatationskatheter, Crimpvorrichtung offen;
- Figur 3B:: die Anordnung gemäss Figur 3A, Crimpvorrichtung geschlossen;
- Figur 4:: Verpackung mit darin in einer Inertfüllung gelagertem ballon-expandierenden Stent, auf einem Dilatationskatheter im gecrimpten Zustand;
- Figur 5A:: Verpackung mit Zugang, mit darin in einer Inertfüllung gelagertem selbst-expandierenden Stent, im ungecrimpten Zustand, mit einem in Position gebrachten Katheter, Crimpvorrichtung offen;
- Figur 5B:: die Anordnung gemäss Figur 5A, im gecrimpten Zustand, Crimpvorrichtung geschlossen;
- Figur 5C:: die Anordnung gemäss Figur 5B, Crimpvorrichtung offen;
- Figur 5D:: die Anordnung gemäss Figur 5C, Aussenschlauch des Katheters partiell über den gecrimpten Stent geschoben;
- Figur 5E:: die Anordnung gemäss Figur 5D, Aussenschlauch des Katheters vollständig über den gecrimpten Stent geschoben; und

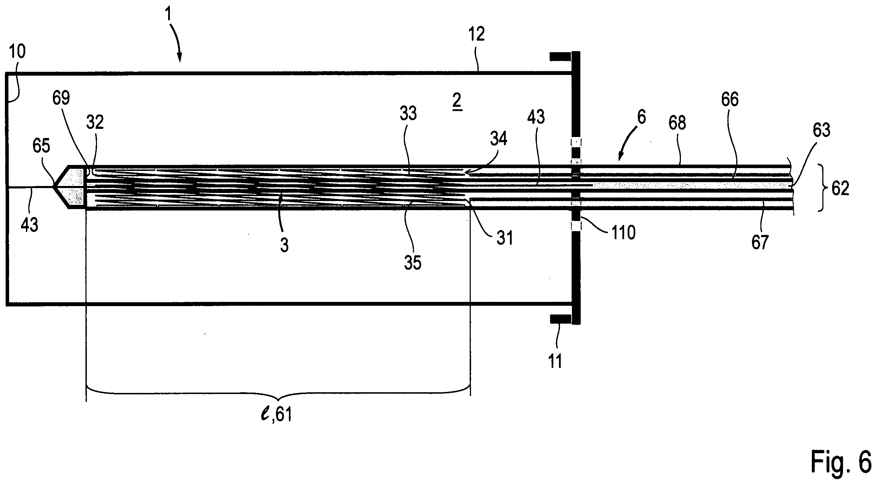

- Figur 6:: Verpackung mit darin in einer Inertfüllung gelagertem selbst-expandierenden Stent, auf einem Katheter montiert, im gecrimpten Zustand.

### Ausführungsbeispiele

Mit Bezug auf die beiliegenden Zeichnungen erfolgt nachstehend die detaillierte Beschreibung der erfindungsgemässen Anordnung, welche aus einem metallischen Stent - als medizinisches Implantat zur Behandlung von Läsionen in Blutgefässen - und einer Verpackung mit einem Innenvolumen, in welchem der Stent geschützt angeordnet ist, besteht.

Für die gesamte weitere Beschreibung gilt folgende Festlegung. Sind in einer Figur zum Zweck zeichnerischer Eindeutigkeit Bezugsziffern enthalten, aber im unmittelbar zugehörigen Beschreibungstext nicht erläutert, so wird auf deren Erwähnung in vorangehenden oder nachfolgenden Figurenbeschreibungen Bezug genommen. Im Interesse der Übersichtlichkeit wird auf die wiederholte Bezeichnung von Bauteilen in weiteren Figuren zumeist verzichtet, sofern zeichnerisch eindeutig erkennbar ist, dass es sich um "wiederkehrende" Bauteile handelt.

### Figur 1A

Der dargestellte Stent **3** ist von herkömmlicher Materialbeschaffenheit und konstruktivem Aufbau; er könnte ballon- oder selbst-expandierend sein. Der Stent **3** hat die Länge **I,** welche sich zwischen dem proximalen Ende **31** und dem distalen Ende **32** erstreckt. Im nicht-gecrimpten Zustand nimmt der Stent **3** den Durchmesser **d** ein, so dass die Stege **33,** welche die Oberfläche **35** besitzen, gitterförmig, weiträumig voneinander beabstandet sind. Durch den röhrchenförmigen Stent **3** verläuft das im Prinzip zylindrische Stentlumen **34.**

### Figur 1B

Der Stent **3** steckt in einer Verpackung **1** und wird hierbei von einer in der Verpackung **1** angeordneten Halterung **13** fixiert, die zunächst ein erstes Stützelement **131** umfasst, an dem das proximale Ende **31** ansteht. Vom zweiten Stützelement **132** wird das distale Ende **32** gefasst. Die Verpackung **1** hat zunächst das Behältnis **12** mit dem Boden **10** und ist am zum Boden **10** gegenüberliegenden Ende mit dem Deckel **11** verschlossen. Das erste Stützelement **131** erstreckt sich trennwandartig über die Querschnittsfläche des Behältnisses **12** und ist dem Deckel **11** zugewandt, wobei ein drittes Stützelement **133** axial den Deckel **11** mit dem ersten Stützelement **131** verbindet. Das zweite Stützelement **132** erstreckt sich ebenfalls trennwandartig über die Querschnittsfläche des Behältnisses **12,** ist aber dem Boden **10** zugewandt. In der Verpackung **1** befindet sich eine inerte Füllung **2,** welche die Oberfläche **35** des Stent **3** schützt. Die dem Stent **3** zugewandten Innenflächen der Verpackung **1** sind inert.

Durch vorgängige Behandlung der Oberfläche **35** wurde deren hydrophile Eigenschaft erhöht. Die aus der Atmosphäre stammenden molekularen chemischen Verunreinigungen auf der Oberfläche **35 -** hauptsächlich Kohlenwasserstoff-Verbindungen - wurden signifikant reduziert, wodurch sich der Kontaktwinkel als Mass der Hydrophilizität eines an der Oberfläche **35** aufsitzenden Wassertropfens verringert.

Die Reduktion der chemischen Verunreinigungen auf der Oberfläche **35** lässt sich vorzugsweise durch Materialabtrag erzielten. Hierfür bieten sich Sputtern als Beschuss mit Ionen, Elektroerosion oder -polieren, Plasmaaktivieren, Laserablation, mechanisch abrasive Verfahren, Trockenätzen oder nass-chemisch Ätzen an. Alternativ wird die Reduktion der chemischen Verunreinigungen auf der Oberfläche **35** durch eine die Topographie der Oberfläche **35** unverändernde Behandlung erzielt. Hierfür kommt die Behandlung mittels Ultraschall, UV-Licht oder Ozon oder eine daraus gebildete Kombinationsbehandlung in Betracht. Gleichermassen eignet sich die Behandlung mit einem Ätzmedium, welches das Stentmaterial selbst nicht angreift, z.B. eine Säurebehandlung der Oberfläche. Bewährt hat sich 95% - 97% Schwefelsäure auf Cobalt-Chrom-Legierung.

### Figuren 2A bis 2C

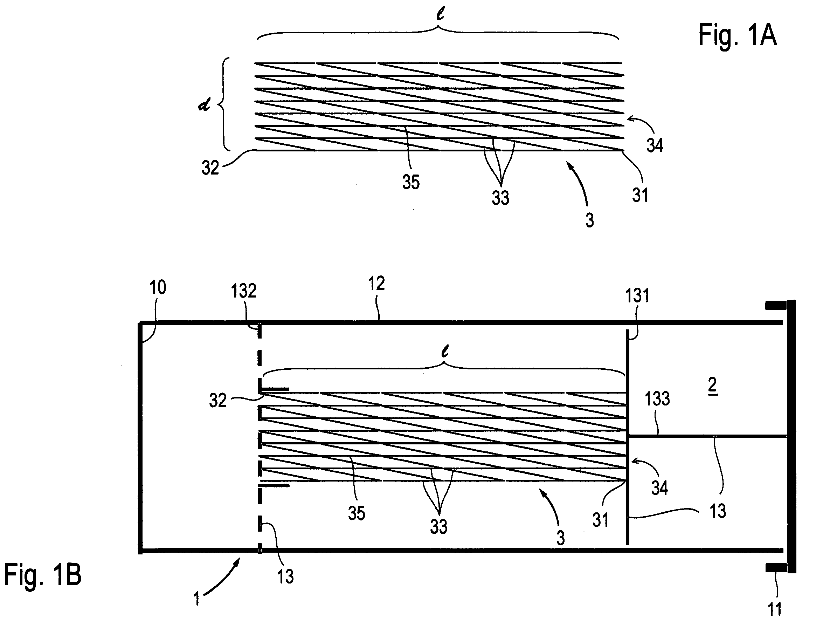

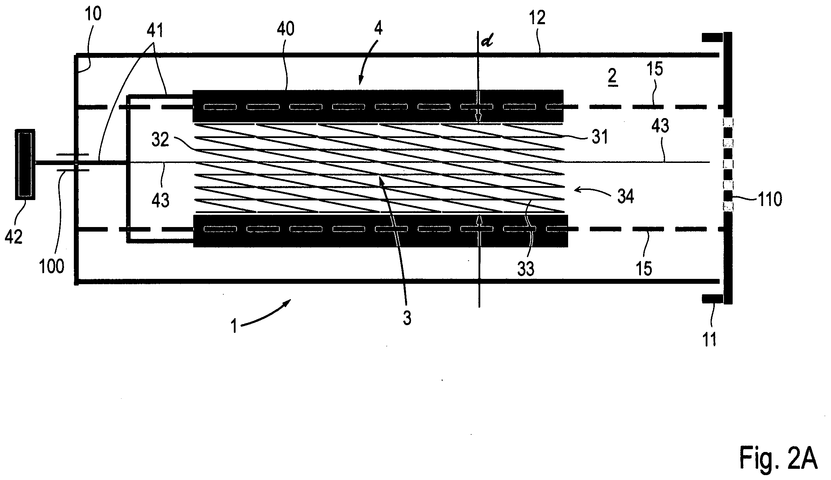

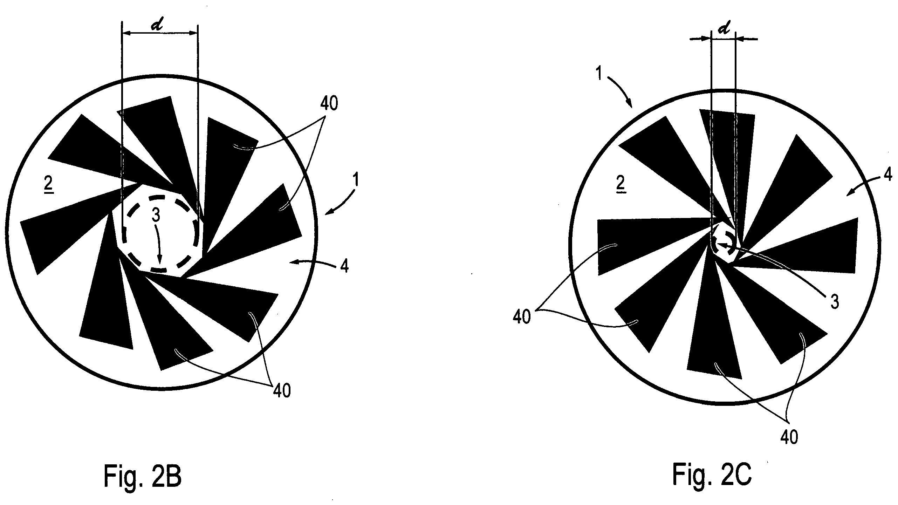

Diese Figurengruppe illustriert die Funktion einer in der Verpackung **1** angeordneten Crimpvorrichtung **4.** Die Crimpvorrichtung **4 ist** zunächst offen, so dass deren Backen **40** eine aufgeweitete Position einnehmen und dabei den in der Verpackung **1** steckenden, expandierten Stent **3** umfassen (s. Figuren 2A, 2B). Die Vorbehandlung des Stent **3** geschieht, wie bereits zur Figur 1B beschrieben. Die Verpackung **1** beinhaltet wiederum die inerte Füllung **2** und die Innenwandung der Verpackung ist inert. Die Backen **40** sitzen auf einer Welle **41,** die axial durch einen Durchgang **100** im Boden **10** nach aussen zu einem betätigbaren Aktivator **42** führt. Das Behältnis **12** wird von sich axial zwischen dem Boden **10** und dem Deckel **11** erstreckenden Achsen **15** durchragt. Zentrisch durch das Behältnis **12** verläuft ein zur Crimpvorrichtung **4** gehörender Führungsdorn **43,** der innerlich des Behältnisses **12** vor einem perforierbaren Zugang **110** am Deckel **11** endet. Bei geschlossener Crimpvorrichtung **4** sind die Backen **40** radial verengt, so dass der Stent **3** im Durchmesser **d** komprimiert ist (s. Figur 2C).

### Figuren 3A und 3B

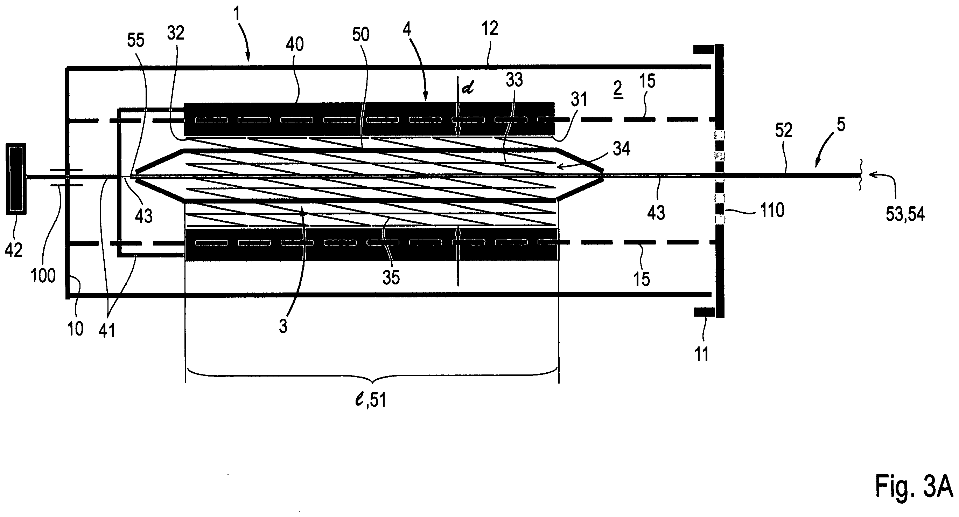

Dieses Figurenpaar basiert auf der Anordnung gemäss Figur 2A, wobei der eingesetzte Stent **3** ballon-expandierend ist und einer Vorbehandlung zur Erhöhung der Hydrophilizität der Oberfläche **35** unterzogen wurde, wie bei Figur 1B dargelegt. Vorausgesetzt werden wiederum eine interte Füllung der Verpackung **1** sowie inerte Eigenschaft deren Innenwandung. Die Backen **40** der Crimpvorrichtung **4** sind zunächst offen (s. Figur 3A). Durch den perforierbaren Zugang **110** im Deckel **11** hat man mit der Spitze **55** voran den auf dem Schaft **52** angeordneten Ballon **50** des Katheters **5** in das Stentlumen **34** eingeführt. Hierbei ist der Führungsdom **43** in das Führungsdrahtlumen **53** des Schafts **52** eingedrungen. Der Schaft **52** hat ferner das kanalartige Dilatationslumen **54,** über welches von aussen, während der Operation, der Ballon **50** durch Auffüllen von innen - z.B. mittels physiologischer Salzlösung - zur Expansion gebracht wird und damit den Stent **3** von innen aufweitet. Der Ballon **50** sitzt mit seinem Stentbereich **51** im Stentlumen **34,** so dass der Stentbereich **51** zumindest im Prinzip die gesamte Stentlänge **I** durchragt, während die sich zuspitzenden Enden des Ballons **50** aus dem proximalen Ende **31** und dem distalen Ende **32** des Stent **3** herausragen.

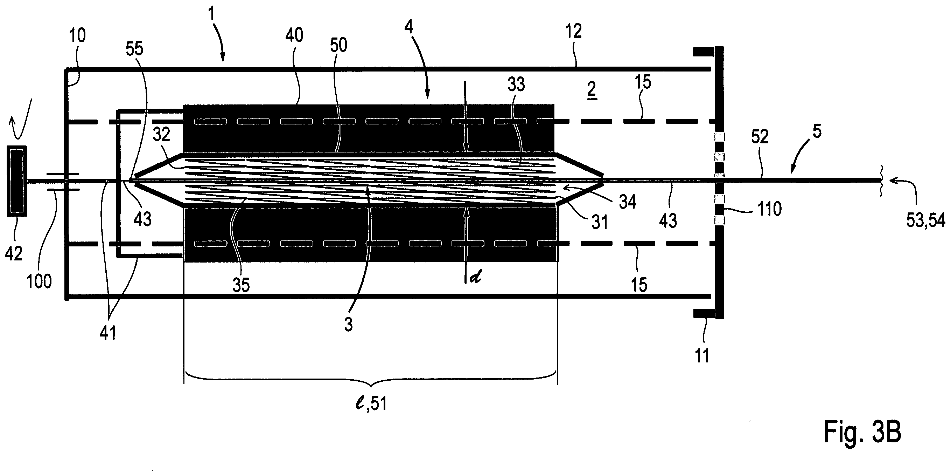

Nach Betätigung des Aktivators **42** durch Drehen, z.B. per Hand, gelangt die Crimpvorrichtung **4** in den geschlossenen Zustand, so dass der Stent **3** in seinem Durchmesser **d** zusammengedrückt wird (s. Figur 3B). Bei dem nun verengten Stentdurchmesser **d** und den komprimierten Backen **40** der Crimpvorrichtung **4** bleibt der Ballon **50** mit seinem Stentbereich **51** innerhalb des Stentlumen **34** in unveränderter axialer Position.

### Figur 4

Alternativ zum Aufbau gemäss den Vorgängerfiguren, wo eine Crimpvorrichtung **4** in der Verpackung **1** integriert ist, hat man hier in der Verpackung **1** einen ballon-expandierenden Stent **3** auf dem Ballon **50** eines Dilatationskatheters **5** im gecrimpten Zustand untergebracht. Dabei ist der Stentdurchmesser **d** verengt und die Stege **33** liegen aneinander gedrängt. Erneut erstreckt sich der Stentbereich **51** des Ballons **50** zumindest im Prinzip über die Stentlänge **I**. Der Führungsdom **43,** der sich vom Boden **10** erstreckt, ist in das Führungsdrahtlumen **53** des Schafts **52** eingedrungen. Die Spitze **55** kommt nahe dem Boden **10** zu liegen. Das Innere der Verpackung **1** ist mit der inerten Füllung **2** versehen, welche die Oberfläche **35** des gemäss Beschreibung zu Figur 1B vorbehandelten Stent **3** schützt. Ferner wird vorausgesetzt, dass die Innenwandung der Verpackung **1** inert ist. Durch den perforierbaren Zugang **110** im Deckel **11** lässt sich der Dilatationskatheter **5** samt gecrimpten Stent **3** und Ballon **50** aus der Verpackung **1** herausziehen.

### Figuren 5A bis 5E

Bei dieser Figurenfolge weist die Verpackung **1** eine integrierte Crimpvorrichtung **4** auf und es kommen ein selbst-expandierender Stent **3** sowie ein Schlauchkatheter **6** zum Einsatz. Zur Crimpvorrichtung **4** gehören wiederum die sich durch den Durchgang **100** im Boden **10** zum Aktivator **42** hin erstreckende Welle **41** und der die Verpackung **1** axial durchragende Führungsdorn **43.** Die Verpackung **1** enthält die inerte Füllung **2** und die Verpackungsinnenwandung ist inert. Innerhalb der Verpackung **1** liegen erneut die Achsen **15.** Die Oberfläche **35** des Stent **3** ist zur Erhöhung der Hydrophilizität vorbehandelt, wie bei Figur 1B erläutert.

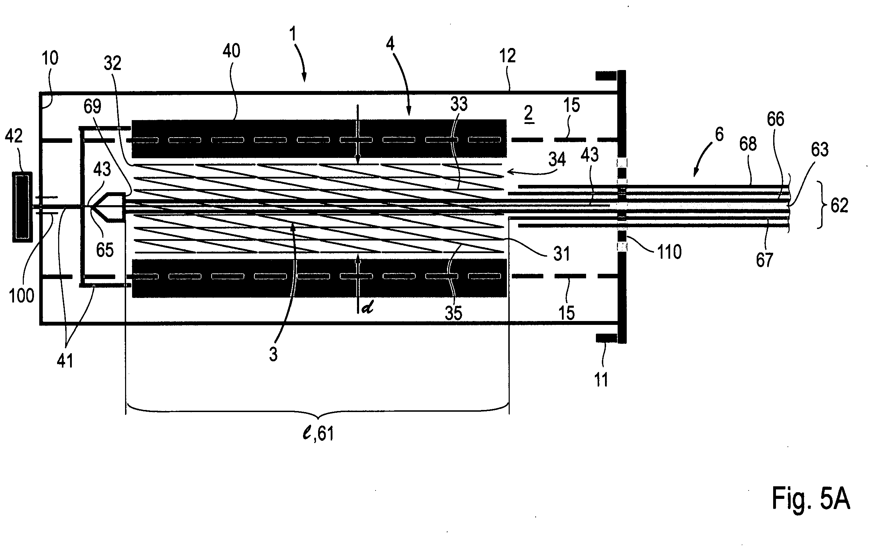

### Figur 5A (Ausgangssituation)

Die Backen **40** der Crimpvorrichtung **4** sind offen, der Stent **3** befindet sich folglich im ungecrimpten Zustand und der Innenschlauch **66** des Schlauchkatheters **6** ist durch den perforierbaren Zugang **110** im Deckel **11** und das Stentlumen **34** soweit eingeschoben, dass die Spitze **65** dem Boden **10** zugewandt aus dem Stent **3** herausragt. Der Führungsdorn **43** ist axial in das Führungsdrahtlumen **63** des Schafts **62** eingedrungen. Der Stützschlauch **67** und der Aussenschlauch **68** sind ebenfalls durch den perforierbaren Zugang **110** eingeschoben, deren freie Enden stehen jedoch vor dem proximalen Ende **31** des Stent **3.** Zwischen dem freien Ende des Stützschlauchs **67** und dem Anschlag **69** an der Spitze **65** erstreckt sich der Stentbereich **61,** der die Stentlänge I aufnehmen kann.

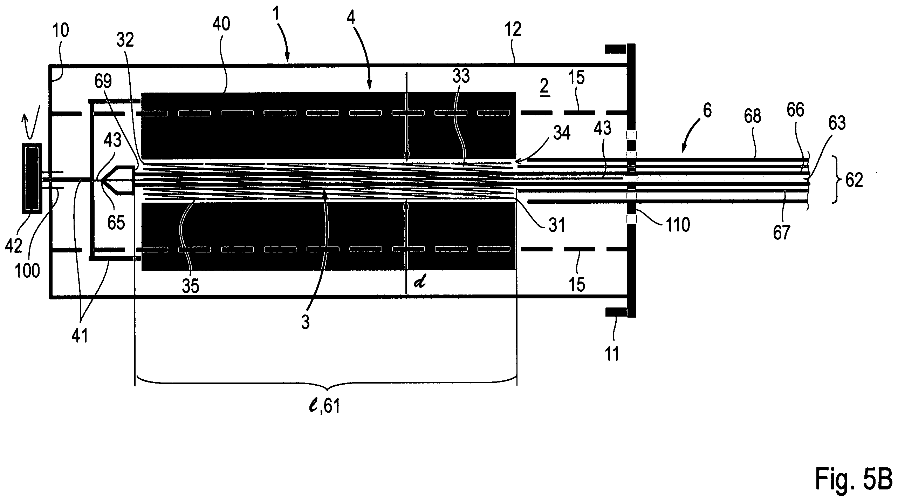

### Figur 5B (1. Fortschritt)

Die Backen **40** der Crimpvorrichtung **4** sind nun geschlossen, so dass die Stege **33** des Stent **3** zusammengedrängt liegen und der Stentdurchmesser **d** verengt ist. Die Betätigung der Crimpvorrichtung **4** erfolgte durch Drehen am Aktivator **42.** Der Schlauchkatheter **6** mit der Spitze **65,** dem Innenschlauch **66,** dem Stützschlauch **67** und dem Aussenschlauch **68** ist in der Positionierung unverändert. Im gecrimpten Zustand wird der Stent **3** heruntergekühlt, um das bei Unterschreiten einer definierten Temperatur selbst-expandierende Verhalten auszuschalten.

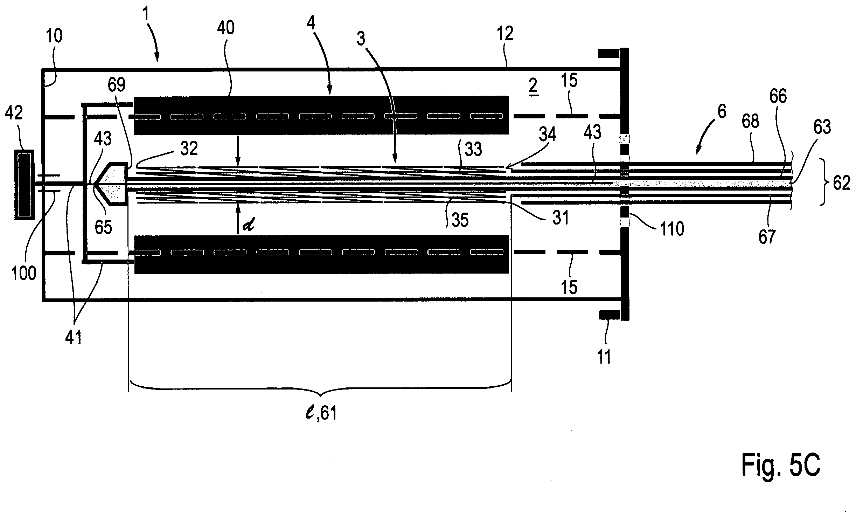

### Figur 5C (2. Fortschritt)

Die Backen **40** der Crimpvorrichtung **4** werden geöffnet, wobei der selbst-expandierende Stent **3** im gecrimpten Zustand mit dem verengten Stentdurchmesser **d** und den verdichteten Stegen **33** durch die zuvor abgesenkte Temperatur verharrt.

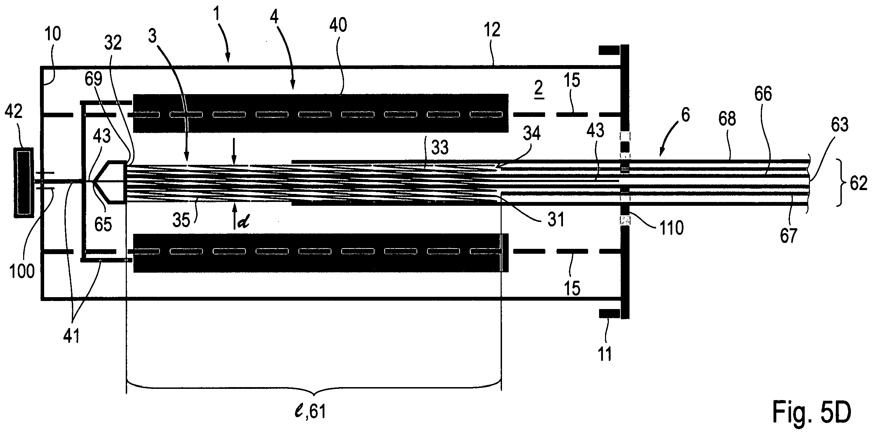

### Figur 5D (3. Fortschritt)

Der im gecrimpten Zustand mit dem verengten Stentdurchmesser **d** verharrende Stent **3** erlaubt vom proximalen Ende **31** ein sukzessives Aufschieben des Aussenschlauches **68** auf den Stent **3** in Richtung des distalen Endes **32.** Der Stützschlauch **67** und die am Innenschlauch **66** angeordnete Spitze **65** bleiben in unveränderter Position. Durch das Vorschieben des Aussenschlauches **68** wird der Stent **3** in gleiche Richtung mitbewegt, wobei der Anschlag **69** das weitere Vorschieben des Stent **3** stoppt.

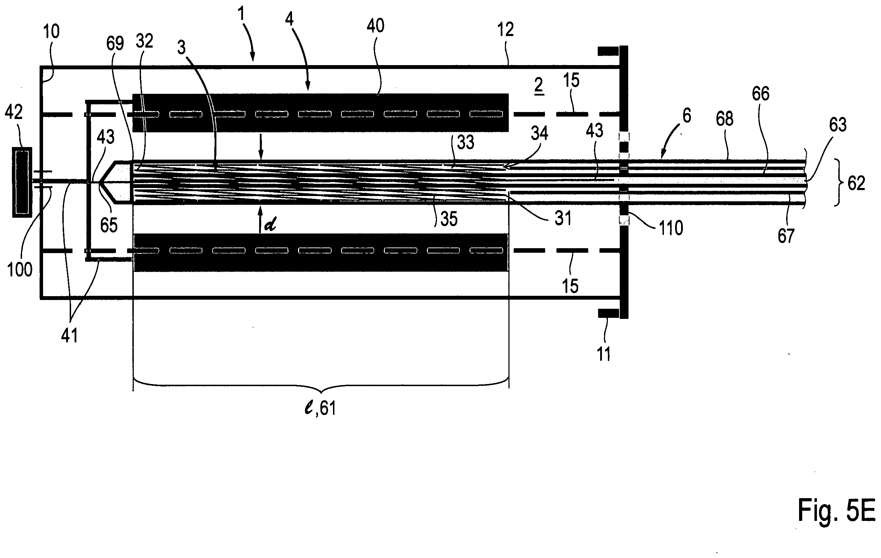

### Figur 5E (4. Fortschritt)

Der Aussenschlauch **68** ist soweit über den gecrimpten Stent **3** aufgeschoben, bis er hinter der Spitze **65** auf den Anschlag **69** trifft und folglich nun den gesamten Stentbereich **61** überdeckt. In diesem Zustand zieht man den Schlauchkatheter **6** mit dem darin gefassten, gecrimpten Stent **3** durch den perforierbaren Zugang **110** aus der Verpackung **1** heraus, um den so vorbereiteten Stent **3** dem Patienten an der zuvor bestimmten Körperstelle zu applizieren.

### Figur 6

Alternativ zum Aufbau gemäss der vorherigen Figurenfolge 5A bis 5E, bei der eine Crimpvorrichtung **4** in der Verpackung **1** integriert ist, hat man nun den selbst-expandierenden Stent **3** ausserhalb der Verpackung **1** gecrimpt und auf den Schlauchkatheter **6** montiert und in die Verpackung **1** eingeführt, so dass sich eine zur Verpackung **1** gehörende Crimpvorrichtung **4** erübrigt. Der Führungsdorn **43** ist in das Führungsdrahtlumen **63** eingefahren. Der Schaft **62** mit Aussenschlauch **68,** Stützschlauch **67** und Innenschlauch **66** durchragen den perforierbaren Zugang **110** im Deckel **11** nach aussen. Der Aussenschlauch **68** steht am Anschlag **69** der Spitze **65** an und übergreift somit den gesamten Stentbereich **61.** Das freie Ende des Stützschlauchs **67** befindet sich vor dem proximalen Ende **31** des Stent **3.** Die weitere Handhabung erfolgt wie im Anschluss an Figur 5E.

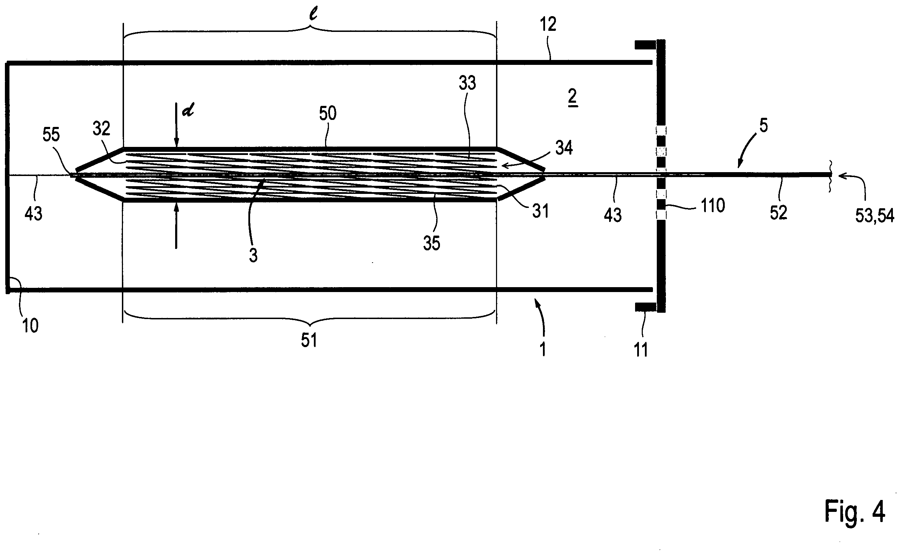

Es wird davon ausgegangen, dass der hier eingesetzte Stent **3** in gleicher Weise vorbehandelt ist, wie bei allen vorherigen Ausführungsbeispielen gemäss den Figuren 1B bis 5E und die Verpackung **1** eine inerte Füllung **2** enthält sowie die Verpackungsinnenwandung inert ist.

## Patentansprüche

1. Anordnung, bestehend aus einem metallischen Stent (**3**) als medizinisches Implantat zur Behandlung von Läsionen in Blutgefässen und einer Verpackung (**1**), mit einem Innenvolumen, in welchem der Stent (**3**) geschützt angeordnet ist, wobei:
a) der Stent (**3**) aufweist:
aa) eine Vielzahl von Stegen (**33**), die miteinander eine Röhrenform bilden;
ab) ein proximales Ende (**31**) und ein distales Ende (**32**), zwischen denen sich ein Stentlumen (**34**) erstreckt; und
ac) eine Oberfläche (**35**) mit hydrophiler Eigenschaft, wobei
b) die aus der Atmosphäre stammenden molekularen chemischen Verunreinigungen, vorrangig Kohlenwasserstoff-Verbindungen, auf der Oberfläche (**35**) durch eine Behandlung signifikant reduziert sind, wodurch sich der Kontaktwinkel als Mass der Hydrophilizität eines an der Oberfläche (**35**) aufsitzenden Wassertropfens im Verhältnis zum Kontaktwinkel vor dieser Behandlung verringert; und
c) der Stent (**3**) in der Verpackung (**1**) inert gelagert ist, um eine natürliche Rekontamination aus der Atmosphäre zu verhindern, **dadurch gekennzeichnet, dass**
d) die Verpackung (1) aus einem Behältnis (12) mit einem Boden (10) und einem Deckel (11) besteht;
e) sich der Boden (10) und/oder der Deckel (11) entfernen lässt: und/oder
f) der Boden (10) und/oder der Deckel (11) einen zu öffnenden Zugang (110) aufweist; so dass
g) sich der Stent (3) aus der Verpackung (1) entnehmen lässt.

2. Anordnung nach Anspruch 1, **dadurch gekennzeichnet, dass**
a) ein Katheter (**5,6**) in der Verpackung (**1**) angeordnet ist und der Stent (3) auf dem Katheter (**5,6**) montiert ist; wobei
b) einem ballon-expandierenden bzw. einem selbst-expandierenden Stent (**3**) komplementär ein Ballonkatheter (**5**) bzw. ein Schlauchkatheter (**6**) zugeordnet ist.

3. Anordnung nach Anspruch 1 oder 2, **dadurch gekennzeichnet, dass**
a) der Katheter (**5,6**) an seinem distalen Ende eine Spitze (**55,65**) hat; und
b) das der Spitze (**55**,**65**) gegenüber liegende proximale Ende eines Schafts (**52**,**62**) des Katheters (**5,6**) den Zugang (**110**) nach ausserhalb der Verpackung (**1**) durchragt.

4. Anordnung nach zumindest einem der vorhergehenden Ansprüche, **dadurch gekennzeichnet, dass** der zu öffnende Zugang (**110**) aus einer durchdringbaren Dichtung oder aus einem perforierbaren Material beschaffen ist.

5. Anordnung nach zumindest einem der vorhergehenden Ansprüche, **dadurch gekennzeichnet, dass**
a) im Boden (**10**) oder im Deckel (**11**) ein Durchgang (**100**) zum Durchlass einer Welle (**41**) vorhanden ist, welche nach innen in die Verpackung (**1**) zu den Backen (**40**) einer integrierten Crimpvorrichtung (**4**) und nach aussen zu einem Aktivator (**42**) zur Betätigung der Crimpvorrichtung (**4**) führt;
b) dem Durchgang (**100**) gegenüberliegend im Deckel (**11**) bzw. im Boden (**10**) der zu öffnende Zugang (**110**) vorhanden ist, der dem Durchlass eines Katheters (**5,6**) dient; und
c) sich ein Führungsdorn (**43**) axial durch die Crimpvorrichtung (**4**) erstreckt, welcher zur Stabilisierung und Positionierung nach dem vollständigen Einführen in ein Führungsdrahtlumen (**53,63**) des Katheters (**5,6**) bestimmt ist.

6. Anordnung nach zumindest einem der vorhergehenden Ansprüche, **dadurch gekennzeichnet, dass** sich in der Verpackung (**1**) Stützelemente (**131-133**) zur Fixierung des Stents (**3**) und/oder des Katheters (**5,6**) und/oder der Crimpvorrichtung (**4**) erstrecken.

7. Anordnung nach zumindest einem der vorhergehenden Ansprüche, **dadurch gekennzeichnet, dass** der gesamte Inhalt der Verpackung (**1**) inert ist und die Verpackung (**1**) eine inerte Füllung (**2**) enthält.

8. Anordnung nach zumindest einem der Ansprüche 1 bis 7, **dadurch gekennzeichnet, dass** der Stent (**3**) für die Anwendung bei Aneurysmen mit einer Ummantelung zu einem Stent Graft ausgebildet ist.

## Claims

1. An arrangement, including a metallic stent (3) as a medical implant for treating lesions in blood vessels, and a packaging (1) with an interior volume, in which the stent (3) is arranged in a protected fashion, wherein
a) the stent (3) comprises:
aa) a multiplicity of webs (33), which together form a tubular shape;
ab) a proximal end (31) and a distal end (32), with a stent lumen (34) extending therebetween; and
ac) a surface (35) with a hydrophilic property, wherein
b) molecular chemical contaminants originating from the atmosphere, mainly hydrocarbons, are significantly reduced on the surface (35) by a treatment, as a result of which, as a measure of the hydrophilicity, the contact angle of a water droplet situated on the surface (35) is reduced compared to the contact angle before this treatment; and wherein
c) the stent (3) is stored in an inert fashion in the packaging (1) in order to prevent natural recontamination from the atmosphere, **characterized in that**
d) the packaging (1) includes a container (12) with a base (10) and a cover (11);
e) the base (10) and/or the cover (11) is removable; and/or
f) the base (10) and/or the cover (11) include an access to be opened (110); such that
g) the stent (3) is removable from the packaging (1).

2. Arrangement according to claim 1, **characterized in that**
a) a catheter (5, 6) is arranged in the packaging (1) and the stent (3) is mounted on the catheter (5, 6), wherein
b) a balloon catheter (5) or a tube catheter (6) is assigned in a complementary fashion to a balloon-expanding or a self-expanding stent (3).

3. Arrangement as according to claim 1 or 2, **characterized in that**
a) the catheter (5, 6) has a tip (55, 65) at its distal end; and
b) the proximal end of a shaft (52, 62) of the catheter (5, 6) opposite the tip (55, 65) protrudes through the access (110) outside of the packaging (1).

4. Arrangement according to any one of the preceding claims, **characterized in that** the access to be opened (110) is made of a penetrable seal or a perforatable material.

5. Arrangement according to any one of the preceding claims, **characterized in that**
a) a passage is formed in the base (10) or in the cover (11) for allowing a shaft (41) to pass (100), which shaft leads to the jaws (40) of an integrated crimping apparatus (4) toward the inside, into the packaging (1), and leads to an activator (42) for actuating the crimping apparatus (4) toward the outside;
b) the access to be opened (110) is opposite the passage (100) in the cover (11) or in the base (10), which access serves to let a catheter (5, 6) pass; and
c) a guide mandrel (43) axially extends through the crimping apparatus (4) and is used for stabilization and positioning after being completely inserted into a guide wire lumen (53, 63) of the catheter (5, 6).

6. Arrangement according to any one of the preceding claims, **characterized in that** support elements (131-133) for fixing the stent (3) and/or the catheter (5, 6) and/or the crimping apparatus (4) extend within the packaging (1).

7. Arrangement according to any one of the preceding claims, **characterized in that** the entire content of the packaging (1) is inert and the packaging (1) contains an inert filling (2).

8. Arrangement according to any one of claims 1 to 7, **characterized in that** the stent (3) is embodied with a cover to form a stent graft for the application in the case of aneurysms.

## Revendications

1. Agencement, constitué d'un stent métallique (**3**) sous forme d'implant médical pour le traitement de lésions dans des vaisseaux sanguins et d'un emballage (**1**), avec un volume interne, dans lequel le stent (**3**) est disposé de manière protégée, dans lequel :
a) le stent (**3**) présente :
aa) une pluralité de baguettes (**33**), qui bâtissent les unes avec les autres une forme tubulaire;
ab) une extrémité proximale (**31**) et une extrémité distale (**32**), entre lesquelles une lumière de stent (**34**) s'étend; et
ac) une surface (**35**) avec une propriété hydrophile, dans lequel
b) les impuretés chimiques moléculaires provenant de l'atmosphère, principalement les composés d'hydrocarbures, sont considérablement réduites sur la surface (**35**) par le biais d'un traitement, moyennant quoi l'angle de contact en tant que mesure de l'hydrophilicité d'une goutte d'eau présente sur la surface (**35**) diminue par rapport à l'angle de contact avant ce traitement; et
c) le stent (**3**) est placé inerte dans l'emballage (**1**), afin d'empêcher une recontamination naturelle provenant de l'atmosphère, **caractérisé en ce que**
d) l'emballage (**1**) est constitué d'un récipient (**12**) avec un fond (**10**) et un couvercle (**11**);
e) le fond (**10**) et/ou le couvercle (**11**) peuvent être retirés: et/ou
f) le fond (**10**) et/ou le couvercle (**11**) présentent un accès à ouvrir (**110**); de sorte que
g) le stent (**3**) puisse être sorti de l'emballage (**1**).

2. Agencement selon la revendication 1, **caractérisé en ce que**
a) un cathéter (**5,6**) est disposé dans l'emballage (**1**) et le stent (**3**) est monté sur le cathéter (**5,6**); dans lequel
b) un cathéter à ballonnet (**5**) ou un cathéter à tube (**6**) est associé de manière complémentaire à un stent (**3**) à expansion automatique ou à expansion par ballonnet.

3. Agencement selon la revendication 1 ou 2, **caractérisé en ce que**
a) le cathéter (**5,6**) possède, au niveau de son extrémité distale une pointe (**55,65**); et
b) l'extrémité proximale d'une tige (**52,62**) du cathéter (**5,6**) située à l'opposé de la pointe (**55,65**) traverse l'accès (**110**) vers l'extérieur de l'emballage (**1**).

4. Agencement selon au moins l'une des revendications précédentes, **caractérisé en ce que** l'accès à ouvrir (**110**) est formé d'un joint pouvant être pénétré ou d'un matériau pouvant être perforé.

5. Agencement selon au moins l'une des revendications précédentes, **caractérisé en ce que**
a) dans le fond (**10**) ou dans le couvercle (**11**) se trouve un passage (**100**) pour le passage d'un arbre (**41**), qui mène vers l'intérieur dans l'emballage (**1**) aux mâchoires (**40**) d'un dispositif de sertissage intégré (**4**) et vers l'extérieur à un actionneur (**42**) pour l'actionnement du dispositif de sertissage (**4**);
b) l'accès à ouvrir (**110**) se trouve à l'opposé du passage (**100**) dans le couvercle (**11**) ou dans le fond (**10**) et sert au passage d'un cathéter (**5,6**); et
c) une broche de guidage (**43**) s'étend axialement à travers le dispositif de sertissage (**4**), qui est conçue pour la stabilisation et le positionnement après l'insertion complète dans une lumière de fil-guide (**53,63**) du cathéter (**5,6**).

6. Agencement selon au moins l'une des revendications précédentes, **caractérisé en ce que** des éléments de support (**131-133**) s'étendent dans l'emballage (**1**) pour la fixation du stent (**3**) et/ou du cathéter (**5,6**) et/ou du dispositif de sertissage (**4**).

7. Agencement selon au moins l'une des revendications précédentes, **caractérisé en ce que** le contenu entier de l'emballage (**1**) est inerte et l'emballage (**1**) contient une charge inerte (**2**).

8. Agencement selon au moins l'une des revendications 1 à 7, **caractérisé en ce que** le stent (**3**) est conçu pour une application sur les anévrismes avec une gaine pour une greffe de stent.
